# EUROPEAN PATENT APPLICATION

(11) **EP 0 602 247 A1**
(43) Date of publication of application: **22.06.1994**
(21) Application number: 93907873.9
(22) Date of filing: 07.04.1993
(51) Int. Cl.: C12P 13/04, C12N 11/04

(54) **METHOD FOR THE PREPARATION OF D-AMINOACIDS OR D-AMINOACIDS DERIVATIVES**

(30) Priority: 10.04.1992 ES 9200775
(71) Applicant: CONTROL Y GESTION INSTRUMENTAL, S.A., E-08007 Barcelona (ES)
(72) Inventor: BENAIGES MASSA, Maria Dolores, E-08007 Barcelona (ES); CAMINAL SAPERAS, Gloria, E-08007 Barcelona (ES); LOPEZ SANTIN, José, E-08007 Barcelona (ES); SERRAT JURADO, Josep Maria, E-08007 Barcelona (ES)
(74) Representative: Carpintero Lopez, Francisco
(86) International application number: ES9300024
(87) International publication number: WO9321336

(57) **Abstract**

The present invention relates to a new method for the preparation of D-aminoacids or derivatives of D-aminoacids from the racemic mixture of the corresponding hydantoin using cells of *Agrobacterium radiobacter* immobilized by inclusion into natural polymer gels.

## Description

This invention refers to a new procedure for the preparation of D-aminoacid or D-aminoacid derivatives from the racemic mixture of the relevant hydantoin, using *Agrobacterium radiobacter* cells immobilized through the insertion of gelled natural polymers.

The use of biocatalysers that use immobilized microorganisms presents significant advantages for a continuous process. The objective is the stereoselective biochemical transformation of a D-hydantoin to obtain the relevant aminoacid-D. The products that can be obtained are of interest for the pharmaceutical industry, for instance the p-hydroxyphenylglycine.

### PRIOR ART

The use of the hydantoinase and/or carbamylase enzymatic system for the transformation of hydantoins is very well known. (Biotechnology: A Comprehensive Treatise. Vol 6a. H. -J. Rehm, G. Reed Eds., Verlag Chemie, Weinheim, 1984).

A large number of microorganisms has been proposed as producers of the above-mentioned enzymatic system, among them: *Pseudomonas, Agrobacterium, Candida, Hansenula, Arthrobacter,* the *Agrobacterium radiobacter* being of special interest due to the fact that it presents a complete enzymatic system featuring both activities.

Among the patents related with this subject those referred to the use of *Agrobacterium radiobacter* to obtain D-phenylglycine or derivatives should be highlighted (Ger. Offen. 2,621,076; Ger. Offen. 2,920,963) as well as the following articles: R. Olivieri et al. "Enzymatic conversion of N carbamyl-D-aminoacides to D-amino acids" **Enzyme Microb. Technol.** 1, 201-204 (1979) and R. Olivieri et al. "Microbial transformation or racemic hydantoins to D- amino acids" **Biotechnol. Bioeng.** 23, 2173-83 (1981). These articles describe a microbial process using free biomass.

In connection with the use of enzymatic systems of other microorganism the following should be mentioned: *Agrobacterium tumefaciens* (EP 0 309 310), *Pseudomonas* (Ger. Offen. 3,018,581), *Candida* (JP 61,177,992), *Hansenula* (JP 61,177,991).

The use of immobilised microorganisms presents significant advantages for operation and separation which are the object of this invention. While the use of *Agrobacterium radiobacter* cells immobilised in gelled polymers has been patented (ES 523.360), this biocatalyser was used for agricultural purposes leaving aside the retention of the hydantoinase and carbamylase enzymatic activities.

On the other hand, the use of other immobilised microorganisms for the bioconversion of hyndantoins - as in the case of EP 0 261 836 which refers to the immobilisation of *Bacillus brevis* - has been described. Furthermore, US patent 4,094,741 should be mentioned. The claims of such patent include, in general terms, the immobilisation of enzymatic activities of a wide number of microorganisms among which neither the *Agrobacterium radiobacter* nor specific methods of immobilisation are mentioned.

### SUMMARY OF THE INVENTION

The object of this invention is a new process for the preparation of D-aminoacids and D-aminoacids derivatives from the racemic mixture of the relevant hydantoin using *Agrobacterium radiobacter* cells immobilised through the inclusion of gelled natural polymers.

The use of immobilised microorganisms enables the reuse of the biocatalyser thus obtaining significant advantages from the point of view both of the separation and the use of a continuous process.

On the other hand, the use of *Agrobacterium radiobacter* cells presents the advantage of the presence of the complete and necessary enzymatic system enabling a quantitative transformation of the D-hydantoin into the desired product.

The stereospecificity of the enzymes involved, acting on the D- form of the hydantoin actuates by displacing the D-L balance of the racemic mixture enabling the quantitative conversion of such racemic mixture into the D-aminoacid or the D-aminoacid derivative.

The immobilisation of *Agrobacterium radiobacter* cells (intact or subject to a disruption process) is obtained through the inclusion of gelled alginate or carragenate formed in the presence of ions Ca²⁺ and/or thermal treatment. The spherical particles of a controlled size so obtained can be subject or not to a later hardness treatment to increase their mechanical endurance.

As a result, a stable biocatalyser is obtained, able to perform the total conversion of the D-L-hydantoin into the relevant D-aminoacid derivative as well as its reuse during a period of at least 22 days.

This invention can be better illustrated in the following experimental example which should not be understood to be of a limitative nature as far as the scope of the invention is concerned. This experimental example is based on the figures shown at the end of this descriptive report, the meaning of which is as follows:
Figure 1 represents the variation of the activity of the hydantoinase as a function of the pH.
Figure 2 represents the variation of the activity of the carbamylase.
Figures 3 and 4 represent the variation of the enzymatic activities as a function of the temperature.
Figures 5 and 6 represent the stability of the free biomass at 45° and 50°C.
Figure 7 represents the stability of the 2 enzymatic activities tested during a period of 22 days at 40ºC.
Figure 8 reflects the performance of the free biomass tested under the same conditions.

### EXPERIMENTAL EXAMPLE

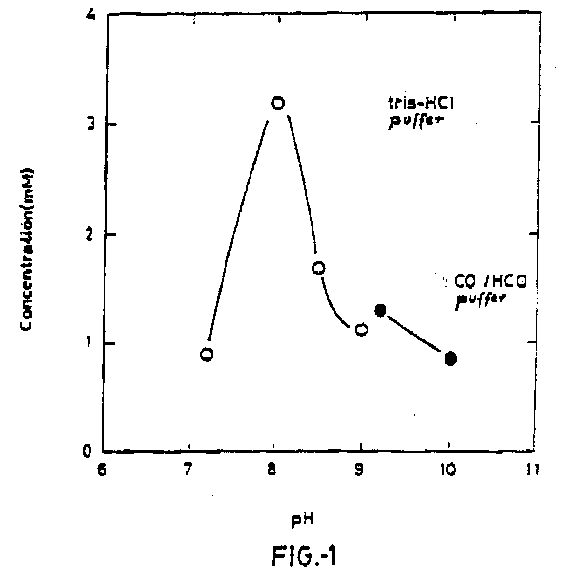

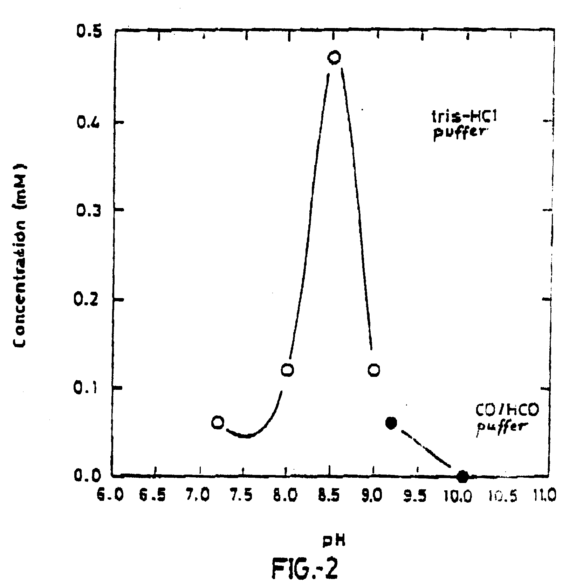

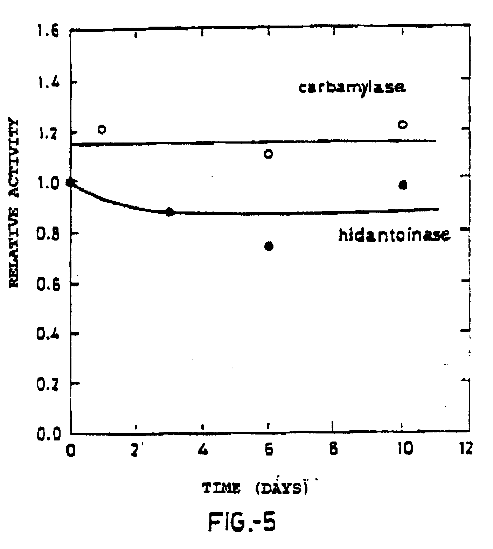

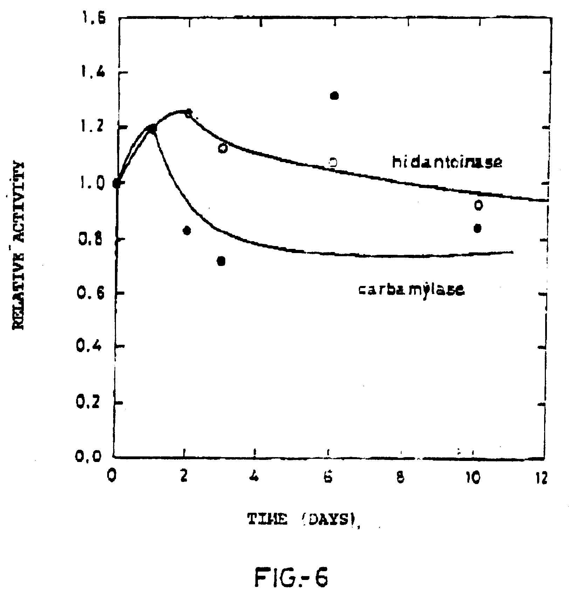

1. - *Agrobacterium radiobacter* cells were used to obtain D-p hydroxyphenylglycine from the relevant hydantoin. The product obtained followed the scheme described below: 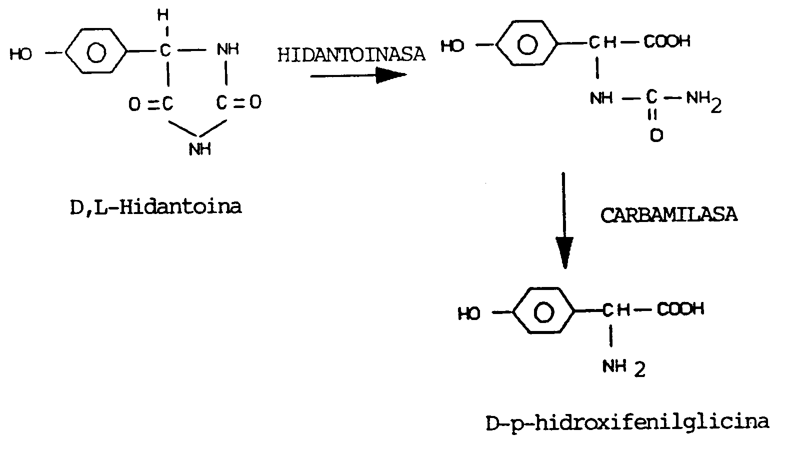
2.- Activity tests
   2.1.- The enzymatic activity of the *Agrobacterium radiobacter* biomass was determined as follows: 1 ml of biocatalyser suspension is added to 11 ml of a hydantoin suspension pH 8 puffer, the final concentration of substrate being 0,02 M. This is incubated at 40ºC during 20 minutes, at a N₂ atmosphere and the D-p-hydroxyphenylglycine obtained through HPLC is analyzed.
      To determine the carbamilase activity, the same activity test is used substituting the hydantoin by the carbamyl derivative.
      The activity of the immobilised biocatalyser was determined through the same procedure, working with 5 ml of biocatalyser.
   2.2.- To determine the best pH of each enzymatic activity, 10 mM solutions of each one of the substrates (hydantoin and carbamyl derivative) were prepared in a tris-HCl puffer or carbonate/carbonic puffer according to the pH desired. A suspension of 1 ml of biomass was added to a solution of substrate of 10 ml and tested at 40º C as indicated in point 2.1.
   2.3.- To determine the best temperature of each activity, the substrates (10 mM) were dissolved in a tris-HCL puffer at an optimal pH in each case (pH 8 for the hydantoinase activity and 8,5 for the carbamylase). The tests of activity were carried out at different temperatures.
   2.4.- The hydantoinase activity is expressed in mmol/l converted in a reaction of 20 min (addition of product + carbamyl derivative). The carbamylase activity is expressed in mmoles/l of p-hydroxyphenylglycine obtained after a reaction of 20 min from the intermediate product.
3.- Immobilisation
   A suspension of *Agrobacterium radiobacter* cells was immobilised in gelled alginate, in accordance with the following procedure:
   Materials: sodic alginate (Protanal LF 10/60) at 40% of the final volume. Biomass of *Agrobacterium radiobacter* at also 21% of the final volume, CaCl₂ at 1.5%.
   20 ml of biomass (0,7 g/ml) were taken and mixed with the sodium alginate solution (2,6 g/46.67 ml). This was magnetically shaken until a homogeneous mixture was obtained. Through a peristaltic pump the gel is carried to a needle (0,5 mm diameter) at 30 cm of a solution of CaCl₂, which is magnetically shaked. The solidification of the drops is produced through the contact of the gel and the Ca²⁺ ions. All the immobilisation steps were carried out at an atmosphere of N₂.
4.- Stability
   4.1.- Stability of the free biomass
      25 ml of biomass were kept at the test temperature and a nitrogen atmosphere while the whole experiment was carried out. At predetermined time intervals, a suspension of 1 ml was taken to perform the activity test.
   4.2.- Stability of the immobilised biomass.
      To determine the stability of the biocatalyser obtained, activity test were periodically performed. The immobilised biocatalyser was kept in sealed containers at the desired temperature and a N₂ atmosphere, with a concentration of hydantoin of 3% (p/V). The biocatalyser was submitted to a complete washing procedure before carrying out the activity tests.
5.- Results
   5.1.- Determination of the operating conditions of each enzymatic activity.
      The variation of the hydantoinase activity as a function of the pH is shown in figure 1 obtaining the maximum variation for pH=8. In the same manner, figure 2 presents the variation of activity of the carbamylase at an optimal pH of 8.5.

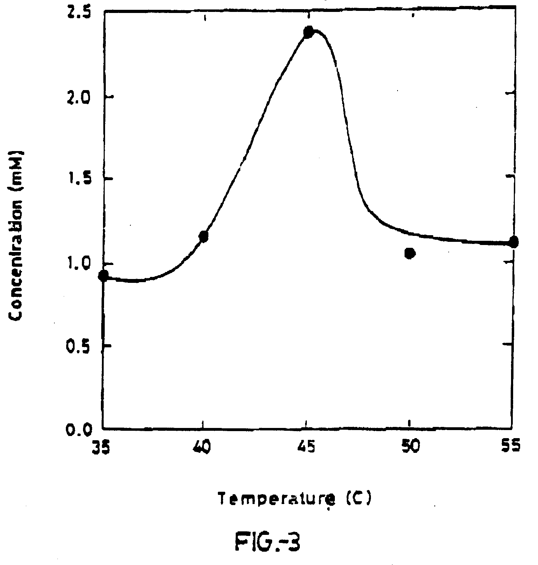

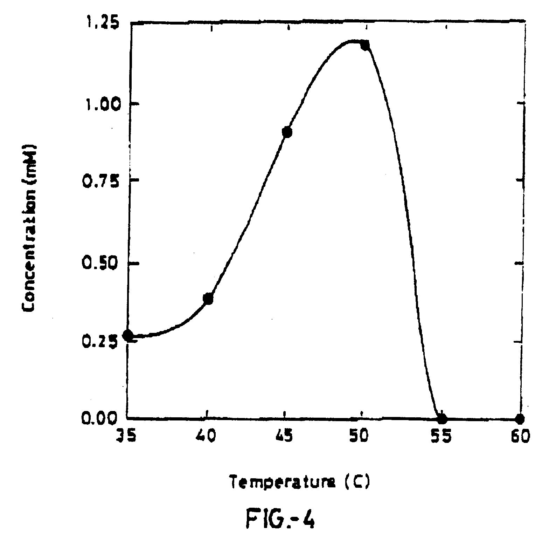

      Figures 3 and 4 show the variation of the enzymatic activities as a function of the temperature. The maximum activity is obtained at 45º C for the hydantoinase and at 50ºC for the carbamylase.
      The stability of the free biomass at 45º and 50ºC is shown in figures 5 and 6. Both enzymatic activities show a high stability during the period of 10 days under consideration although the carbamylase activity at 50º C suffers a loss of approximately 20%.
      The data obtained in connection with the activities and stability enable to reach the conclusion that the operating temperature should be between 45º and 50ºC and the optimal pH between 8 and 8.5. However, the lesser carbamylase activity present in the biomass - when compared with the hydantoinase - makes it advisable to propose the use of values closer to the optimal ones for this activity (pH 8.5 and a temperature of 50ºC).
   5.2.- Operation with an immobilised biocatalyser
      The results obtained from the immobilisation show a retention between 50% and 80% of the activity of the immobilised catalyser (it should be taken into account that the measurement of the activity reflects not only the intrinsical enzymatic activity but also the diffusional limitations in the case of the immobilised biocatalyser).

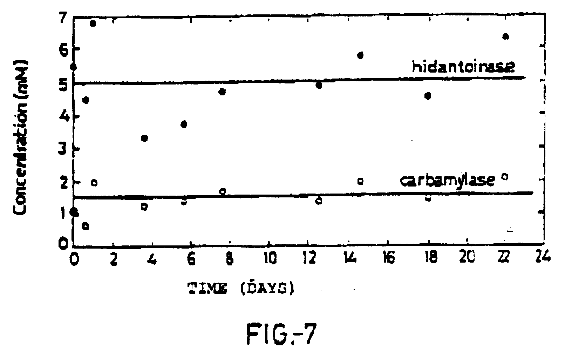

The biocatalyser particles obtained showed a stability of 100% as far as the 2 tested enzymatic activities during a period of 22 days at 40ºC were concerned. (see figure 7).

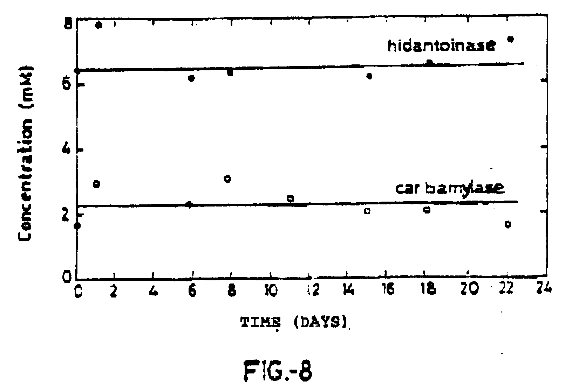

This performance is identical to that of the free biomass tested under the same conditions (see figure 8).

The immobilised biocatalyser was, therefore, able to keep, under the operating conditions, the enzymatic activities of the *Agrobacterium radiobacter* cells and to perform the specific transformation of the D-hydantoin into the D-p-hydroxyphenylglycine.

## Claims

1. Procedure for the preparation of D-aminoacids or derivatives of D-aminoacids from the relevant hydantoin characterized in that such preparation is made by using immobilised microorganisms containing the hydantoinase and carbamylase enzymatic activities.

2. Procedure according to claim 1, characterized in that the microorganisms are immobilised through inclusion into gelled natural polymers (for instance, alginate, carrageenate).

3. Procedure according to claim 2, characterized in that the microorganism is *Agrobacterium radiobacter*.

4. Procedure according to claim 3, characterized in that the product obtained from the hydantoin racemic mixture is the D-p-hydroxyphenylglycine.

5. Procedure according to any of the claims 1, 2, 3 or 4, characterized in that the work is carried out under a reducer environment either an atmosphere of N₂ or in the presence of chemical reducers such as Na₂S, among others.
